# EUROPÄISCHE PATENTSCHRIFT

(11) **EP 2 256 483 B1**
(45) Veröffentlichungstag und Bekanntmachung des Hinweises auf die Patenterteilung: **16.01.2013**
(21) Anmeldenummer: 10163974.8
(22) Anmeldetag: 26.05.2010
(51) Int. Cl.: G01N 21/51, G01N 21/90

(54) **Verfahren und Vorrichtung zur Bestimmung einer Schaumdichte**
Method and device for determining the thickness of foam
Procédé et dispositif destinés à la détermination d'une densité de mousse

(30) Priorität: 26.05.2009 DE 102009022691
(43) Veröffentlichungstag der Anmeldung: 01.12.2010
(73) Patentinhaber: Krones AG, 93073 Neutraubling (DE)
(72) Erfinder: Piana, Stefan, 93096 Köfering (DE)
(74) Vertreter: Grünecker, Kinkeldey, Stockmair & Schwanhäusser

(56) Entgegenhaltungen:
- EP-A1- 0 544 428
- WO-A1-2005/003758
- DE-A1-102004 054 859
- DE-A1-102007 004 346

## Beschreibung

Die Erfindung betrifft ein Verfahren zur Bestimmung der Schaumdichte von Schaum nach dem Oberbegriff des Anspruchs 1 sowie eine Vorrichtung hierzu nach dem Oberbegriff des Anspruchs 4.

In Getränkeabfüllanlagen entsteht bei der Abfüllung von insbesondere kohlesäurehaltigen Getränken in Getränkebehältnisse wie z.B. Flaschen häufig eine Schaumschicht. Je nach Getränkeart kann mehr oder weniger Schaum entstehen. Insbesondere bei der Abfüllung von Bier entsteht häufig viel Schaum. In der Regel vergeht einige Zeit bis sich der Schaum gesetzt hat, d. h. sich die im Schaum gebundene Flüssigkeitsmenge gelöst hat. Zur Kontrolle der Flüssigkeitsmenge in dem Getränkebehältnis wird hierzu der Füllpegel gemessen. Wegen des sich bildenden Schaums ist es aber schwierig, wenn nicht gar unmoglich, eine genaue Bestimmung des Füllpegels unmittelbar nach dem Befüllen durchzuführen, weil man dazu an sich warten muss, bis sich der Schaum gesetzt hat. Allein aufgrund der Abmessungen des Schaums eine Bestimmung der in dem Schaum gebundenen Flüssigkeit durchzuführen ist meist fehlerbehaftet, da der sich bildende Schaum mit unterschiedlicher Dichte entsteht, so dass die gebundene Flüssigkeitsmenge nicht nur von der Art des Getränkes abhängig ist, sondern auch von Behältnis zu Behältnis variiert.

Wenn sauerstoffempfindliche Getränke wie z.B. Bier abgefüllt wird, ist es ausserdem üblich, kurz vor dem Verschliesser einen feinen, sterilen Wasserstrahl in die Füllmündung einzuspritzen (sog HDE -Verfahren). Der dabei erzeugte Schaum verdrängt dann den noch oberhalb des Füllpegels vorhandenen Sauerstoff. Zur Überprüfung bzw. Kontrolle der einwandfreien Funktion wäre es ebenfalls wünschenswert, etwas über die dabei entstehende Schaumdichte zu wissen.

Mit gängigen Methoden, wie Kurzwelle (HF), IR-Ablenkung, IR-Absorption lässt sich die Flüssigkeitsmenge in dem Schaum nicht bestimmen. Diese Verfahren haben den Nachteil, unterschiedliche Schaumgefüge nicht erfassen zu können.

Die WO 2005 / 003758 A1 beschreibt eine Vorrichtung zur Vermessung der Blasengröße eines Schaums in einem Messgefäß, wie beispielsweise ein Messbecher, durch Beleuchten des Schaums mit einem Stroboskop oder dergleichen und Abbilden des Schaums mittels einer CCD-Kamera.

Die DE 10 2004 054 859 A1 beschreibt ein Verfahren und eine Vorrichtung zur optischen Schaumkontrolle in Flaschenhälsen, wobei der Bereich des Flaschenhalses einschl. des Verschlusses mittels einer Infrarotlichtquelle flächig beleuchtet und im Durchlicht von einer Kamera abgebildet wird.

Die EP 0544428 A1 beschreibt ein Verfahren und eine Vorrichtung zur Beurteilung der Schaumqualität eines Getränks nach der Abfüllung. Das Getränk wird hierzu in ein Becherglas gegossen, so dass es seitlich beleuchtet und von einer Kamera im Durchlicht abgebildet werden kann. Hierzu wird eine Lichtquelle zur gleichförmigen Beleuchtung des Becherglases und seines Inhalts vorgeschlagen.

Die DE 10 2007 004346 A1 beschreibt eine Vorrichtung zur optischen Charakterisierung von Proben, wobei diese aus mehreren seitlichen Richtungen, und optional von oben, beleuchtet und von einer Kamera abgebildet werden. Beschrieben sind beispielsweise eine seitliche Lichtquelle zum Erzeugen von Durchlicht und eine näherungsweise koaxial mit der Kamera angeordnete Lichtquelle zum Erzeugen von Auflicht. Ferner wird ein Laser beschrieben, der schräg zur Blickrichtung der Kamera ausgerichtet ist, so dass der Laserstrahl nach dem Durchdringen der Probe auf einen Schirm fällt, wo er von der Kamera abgebildet werden kann.

Es sind zwar Verfahren zur Messung von Schaum bekannt, mit diesen lassen sich aber nur Zerfallsraten der Schäume bestimmen. Bei einem dieser Verfahren wird ein Lichtstrahl in den Schaum emittiert, wobei das auf der gegenüberliegenden Seite aus dem Schaum ausfallende Licht von einer Messeinrichtung erfasst wird. Lichtstrahl und Messeinrichtung rotieren während des Verfahrens um die Schaumschicht. Mit diesem Verfahren ist indes keine genaue Bestimmung der Schaumdichte möglich.

Es ist deshalb Aufgabe der Erfindung, ein Verfahren und eine Vorrichtung vorzuschlagen, mit dem die gebundene Flüssigkeitsmenge in dem Schaum erfasst werden kann, um z.B. den nach dem Setzen des Schaums resultierenden Füllpegel zu berechnen bzw allgemein Schaumbildungen überwachen zu können.

Das Verfahren zur Lösung dieser Aufgabe weist die Merkmale des Anspruchs 1 auf. In den unmittelbar nach dem Einfüllen der Flüssigkeit in das Behältnis bzw. vor dem anschliessenden Verschliessen gezielt erzeugten Schaum sich ausbildende Schaum wird ein Lichtstrahl eingestrahlt. Dieser Lichtstrahl dringt in den Schaum ein und wird dabei aufgeweitet. Zur Bestimmung der Schaumdichte wird die Kontur eines vom Lichtstrahl in dem Schaum erzeugten Lichtflecks bestimmt, wobei die Kontur Rückschlüsse über die Schaumdichte zulässt.

Das Verfahren nutzt hierbei den Effekt aus, dass der Schaum eine Streuung des Lichtstrahls hervorruft. Dabei bewirkt eine unterschiedlich hohe Schaumdichte eine unterschiedlich stark ausgebildete Streuung, d.h. Aufweitung des Strahls. Bei einem hochdichten Schaum, der eine hohe Flüssigkeitsmenge bindet, erfolgt eine starke Streuung der Lichtstrahlen. Umgekehrt ist die Streuung geringer, wenn der Schaum weniger Flüssigkeit bindet, der Schaum also weniger dicht ist.

Die hier beobachtete Streuung des Lichtes wird auch als Tyndall-Effekt bezeichnet. Dieser Effekt tritt auf, wenn Partikel in einer Flüssigkeit oder in einem Gas suspendiert sind, wobei die Größe der Partikel mit der Wellenlänge des Lichts vergleichbar ist (ca. 100 bis 1000 nm). Das Licht wird an den Partikeln, hier eine oder mehrere Blasenwände, gebrochen, wobei durch diese Streuung des Lichts Lichtstrahlen seitlich aus dem Medium herausgestreut werden. Die Streuung bewirkt, dass der Lichtstrahl auch von der Seite her sichtbar ist.

Ferner wird die Kontur des Lichtflecks von außerhalb des Behältnisses bestimmt und mit abgespeicherten Daten verglichen. Für gewöhnlich werden die schäumenden Getränke in durchsichtige Glas- oder Kunststoffflaschen abgefüllt. Der Lichtstrahl, der von einem handelsüblichen, insbesondere fokussierenden Laser im Leistungsbereich weniger mw erzeugt werden kann, kann somit von außerhalb des Behältnisses auf den Schaum gerichtet werden. Die Messeinrichtung, die die Kontur des Lichtflecks erfasst, ist somit ebenfalls außerhalb der Flasche anzuordnen.

Um die Genauigkeit der Bestimmung der Schaumdichte weiter zu erhöhen, kann die Dichte des Schaums mittels mehrer Lichtstrahlen, insbesondere Lichtbündel, bestimmt werden. Es ist bekannt, dass Schaum über seine Ausdehnung keine konstante Dichte aufweist, so dass bei einer singulären Messung Fehler auftreten können. Die Lichtstrahlen werden derart in den Schaum eingestrahlt, dass die sich bildenden Lichtflecke sich nicht überschneiden und deren Kontur klar und deutlich erkennbar ist. Alternativ können auch unterschiedlich gepulste Laser eingesetzt werden, mit denen auch überlappende Lichtflecken erkennbar sind und daraus die Dichte bestimmt werden kann.

Zusätzlich werden die durch die Lichtstrahlen hervorgerufenen Lichtflecken mittels wenigstens einer Messeinrichtung bestimmt. Die Messeinrichtung wird auch für die Bestimmung der Schaumhöhe in der Getränkeflasche benutzt, so dass diese bereits die gesamte Schaumhöhe erfasst. Mit einer Messeinrichtung ist es je nach Ausrichtung der Lichtstrahlen möglich, mehrere Lichtflecken zu erfassen und die Dichte in einem jeweiligen Bereich des Lichtstrahls zu bestimmen. Es können aber auch mehrere Lichtstrahlen so ausgerichtet sein, dass mehrere Messeinrichtungen notwendig sind, um alle Lichtflecken zu erfassen.

Mittels der dabei ermittelbaren Flüssigkeitsmenge im Schaum lässt sich somit der zu erwartende Füllpegel in dem Behältnis bestimmen oder aber allgemein die Qualität bzw. Dichte von Schaum erfassen.

Eine Vorrichtung zur Lösung der genannten Aufgabe weist die Merkmale des Anspruchs 4 auf. Danach ist die Messeinrichtung so ausgebildet, dass sie die Kontur eines im Schaum vom Lichtstrahl erzeugten Lichtflecks erfassen kann. Die Vorrichtung weist hierfür eine Lichtquelle und eine Messeinrichtung auf, die im Bereich der zu befüllenden Behältnisse angeordnet sind. Die Kontur läßt Rückschlüsse auf die Schaumdichte zu, wodurch sich mit einfachen Mitteln die Dichte des Schaums bestimmen läßt.

Ferner weist die Vorrichtung einen Rechner, konfiguriert zur Berechnung der in dem Schaum gebundenen Flüssigkeitsmenge durch Vergleich von gespeicherten und durch die Messeinrichtung gemessenen Daten der Kontur des Lichtflecks auf.

Gemäß einer vorteilhaften Weiterbildung der Erfindung ist die Messeinrichtung, insbesondere eine Kamera, seitlich vom Lichtstrahleingang angeordnet, um den Lichtfleck von der Seite her zu messen. Die Brechung ruft eine Streuung des Lichtstrahls hervor, wodurch der Lichtstrahl seitlich zu seiner Längsachse in dem Schaum sichtbar wird. In Abhängigkeit des resultierenden Streustrahls von der Schaumdichte variiert der Lichtfleck hinsichtlich Umriß, Größe und Helligkeit auf dem Schaum. Mittels der Kamera lässt sich die Kontur des Lichtstrahls bzw. der Umriss des Streustrahls leicht erfassen. Kontur bedeutet hier also die Abnahme der Helligkeit von der Strahlmitte nach beiden Seiten quer zur Strahlrichtung entlang des Strahls sowie die absoluten Helligkeitswerte. In einer vereinfachten Auswertung wäre das beispielsweise das Verhältnis von Länge zu Breite des Umrisses des Streustrahls.

Nach einem weiteren vorteilhaften Ausführungsbeispiel ist vorgesehen, daß der Lichtstrahl von einem vorzugsweise gepulsten Laser oder einer ähnlichen fokussierenden Lichtquelle erzeugt wird. Ein Laser emittiert einen scharfen, gebündelten Lichtstrahl, was bei der Konturmessung des Lichtflecks auch ohne weitere optische Bündelung zu einem guten Ergebnis führen kann.

Vorteilhaft kann der Laser auf die Frequenz der Kamera getriggert sein. Somit ist es möglich, nicht nur Einzelbilder des Schaums aufzunehmen, sondern auch mehrere Bilder hintereinander bzw. ein fortlaufendes Bild in Form eines Films.

Nach einer weiteren vorteilhaften Ausgestaltung der Erfindung sind mehrere Lichtquellen und Messeinrichtungen seitlich des Behältnises angeordnet. Wegen der selbst in einer einzigen Schaumformation unterschiedlichen Schaumdichte ist es für die Genauigkeit der Messung nützlich, mehrere Lichtquellen derart anzuordnen, dass unterschiedliche Bereiche des Schaums messbar sind. Vorteilhaft sind dabei dann die Lichtquellen übereinander entlang der Höhe des Schaums angeordnet. Alternativ dazu können die Lichtquellen auch in einer Ebene angeordnet sein, so dass der Schaum von jeweils zwei oder mehr Seiten mit einem Lichtbündel bestrahlt wird. Die Kamera hingegen ist für gewöhnlich derart ausgebildet, dass ein größerer Bereich des

Schaums erfasst wird, womit es möglich ist, mit einer Kamera mehrere übereinander und/oder nebeneinander angeordnete Lichtquellen und deren erzeugte Lichtflecke zu erfassen. Zur genauen Bestimmung der Kontur der Lichtflecke werden dann mehrere auf einem Bild festgehaltene Lichtflecke separiert.

Die Messeinrichtung ist seitlich und/oder parallel zu der Längsachse des Lichtstrahls angeordnet. Da der Lichtfleck seitlich zur Längsachse des Lichtstrahls aus dem Schaum austritt, ist die Messeinrichtung seitlich zur Längsachse des Lichtstrahls angeordnet. Hierbei spielt es keine Rolle, ob diese in ihrer Ausrichtung senkrecht zum Lichtstrahl angeordnet ist. Es ist lediglich darauf zu achten, dass die Messeinrichtung den Lichtfleck bzw. die Kontur des Lichtflecks gleichbleibend erfasst.

Nach einer bevorzugten Weiterbildung umfasst der Rechner einen Speicher, in dem Daten über das Behältnis und die Flüssigkeit, insbesondere Abmessung und Materialstärke des Behältnisses, vorzugsweise des Flaschenhalses, sowie Qualität der Flüssigkeit gespeichert sein können. Diesen Daten und Informationen werden vor dem Befüllen der Behältnisse ermittelt und in dem Speicher abgelegt. Aufgrund der exogenen Messung, also von außerhalb des Behältnisses, beeinflußt eine Krümmung und die Materialstärke der Behältniswand die Messung des Lichtflecks, was dann bei entsprechender vorheriger Abspeicherung bei der Ergebnisermittlung berücksichtigt werden kann. Ebenso läßt sich über die gemessene Höhe des Schaums und das bekanntes Volumen des Behältnisses in diesem Bereich das Volumen der Schaumschicht ermitteln. Der Rechner weist eine Bildverarbeitungssoftware zur Bestimmung der Kontur des Lichtflecks auf. Die von der Kamera gelieferten Daten werden derart von der Bildverarbeitung aufbereitet, daß die genaue Größe des Lichtflecks in beispielsweise einer Flächeneinheit bestimmbar ist. Das gleiche ist ebenso gültig für die Berechnung der Schaumhöhe, die mit der Bildverarbeitung möglich ist, sowie der Einfüllmenge. Mit einer Bildverarbeitungssoftware sind somit Rückschlüsse auf die Schaumdichte möglich. Im Falle sich überlappender Lichtflecke oder zeitlich nach einander erfolgter Aufnahmen, sind diese ebenfalls mithilfe der Bildverarbeitungssoftware auswertbar.

Weiterhin ist es vorteilhaft möglich, die Vorrichtung in bestehende Kamera-Füllhöhenkontroll-Vorrichtungen zu integrieren. Bestehende Kamerasysteme, die für die Füllhöhenkontrolle bereits in Abfüllanlagen installiert sind, lassen sich somit mit wenig Aufwand einfach nachrüsten.

Ein bevorzugtes Ausführungsbeispiel der Erfindung wird nachfolgend anhand der Zeichnung näher erläutert. In dieser zeigen:
Fig. 1 eine Seitenansicht eines oberen Bereichs eines Getränkebehältnisses mit seitlich angeordneten Lichtquellen,
Fig. 2 einen Draufsicht auf das Getränkebehältnis mit seitlich angeordneten Lichtquellen und einer Messeinrichtung, und
Fig. 3 eine Prinzipskizze zur Erläuterung der Erfindung.

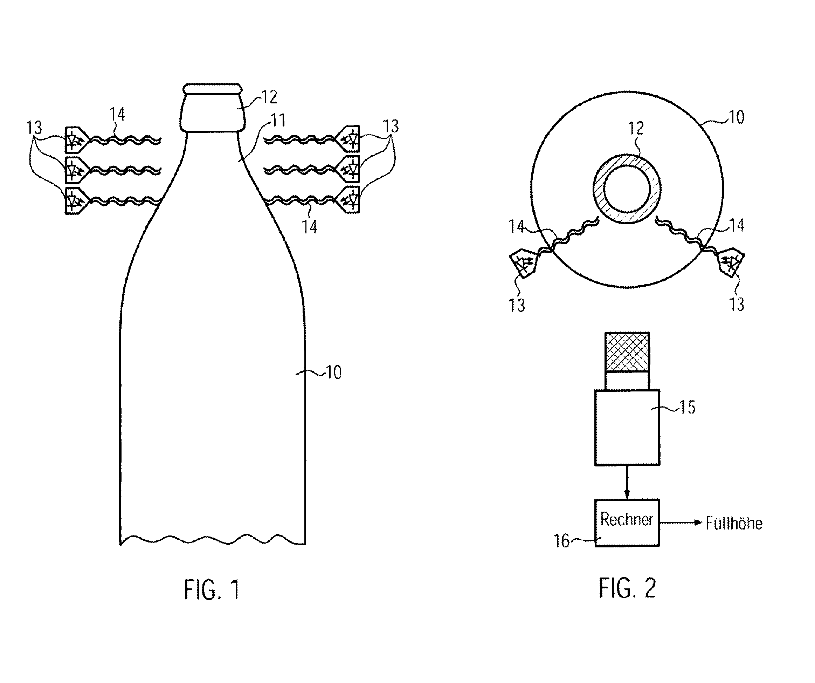

In Fig. 1 ist ein oberer Bereich eines Getränkebehältnisses dargestellt. Hierbei handelt es sich um eine Getränkeflasche 10 mit einem sich konisch verjüngenden Flaschenhals 11. Der Flaschenhals 11 mündet in einem sich verdickenden Öffnungsbereich 12. Bei der Getränkeflasche 10 handelt es sich um eine handelsübliche Enghalsflasche, wie sie regelmäßig in der Getränkeindustrie eingesetzt wird, insbesondere in der Bierindustrie.

In dem Bereich des Flaschenhalses 11 sind bei dem dargestellten Ausführungsbeispiel seitlich mehrere Lichtquellen in Form von Lasern 13 angeordnet. Dabei liegen jeweils zwei Laser 13 in einer Ebene. Insgesamt sind sechs Laser 13 in drei übereinander angeordneten Ebenen angeordnet. Somit wird der Flaschenhals 11 zumindest in einem Bereich unterhalb des Öffnungsbereichs 12 von Lasern 13 umgeben, die entlang einer Längsachse der Getränkeflasche 12 angeordnet sind.

Die Laser 13, die in einer Ebene angeordnet sind, sind am Umfang versetzt zueinander positioniert. Das bedeutet, dass die jeweils emittierten Lichtbündel 14 der Laser 13 nicht axial zueinander verlaufen. Vielmehr liegen deren Achsen in der entsprechenden Ebene im gezeigten Ausführungsbeispiel um ca. 120° versetzt zueinander. Eine Anordnung zweier in einer Ebene angeordneter Laser 13 geht aus der Fig. 2 hervor.

Fig. 2 zeigt eine Draufsicht auf die Getränkeflasche 10 mit zwei seitlich angeordneten Lasern 13. Zusätzlich ist eine Messeinrichtung 15 in Form einer Kamera seitlich zur Getränkeflasche 10 in der Ebene der beiden Laser 13 angeordnet. In der Regel ist die Messeinrichtung 15 derart zur Getränkeflasche 10 positioniert, dass sie den gesamten Bereich des Flaschenhalses 11 abdeckt. Das bedeutet, dass die übereinander angeordneten Ebenen der Laser 13 mittels einer einzigen Messeinrichtung 15 erfasst werden. Einer Messeinrichtung 15 sind demnach mehrere parallel zueinander und über- und untereinander angeordnete Laser angeordnet. Alternativ hierzu kann aber auch jedem Laser 13 bzw. einer Ebene mit Lasern 13 eine Messeinrichtung 15 zugeordnet.

In Fig. 2 ist außerdem ein Rechner 16 dargestellt , der einen Speicher aufweist, in dem Daten über die Getränkeflasche 10 und das Getränk gespeichert sein können. Zur Berechnung werden z. B. Daten über die Abmessung der Flasche 10, insbesondere des Flaschenhalses, und der Wandstärke der Flasche 10 gespeichert. Auch können eventuell Daten über das Getränk selbst gespeichert werden, um mithilfe von Getränkeeigenschaften eine genauere Inter- bzw. Extrapolation über die gesamte Höhe und damit die Erstellung eines vertikalen Dichteprofils zu ermöglichen. Die gespeicherten Daten können auf Erfahrungswerten, Versuchen oder vorherigen Messungen beruhen. Des weiteren weist der Rechner eine Bildverarbeitungssoftware auf. Mittels der Bildverarbeitung ist die Kontur des Lichtflecks 17a, 17b bestimmbar.

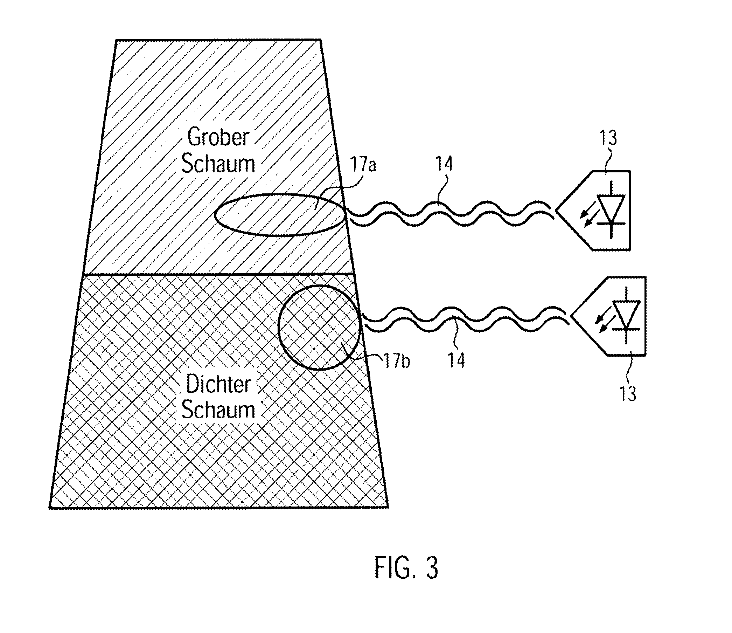

Nachfolgend wird das erfindungsgemäße Verfahren anhand der Fig. 3 näher erläutert: Die beim Befüllen im Bereich des Flaschenhalses 11 entstehende Schaumschicht enthält eine Flüssigkeitsmenge, die, nachdem der Schaum sich gesetzt hat, den Pegel in der Getränkeflasche 10 steigen lässt. Um bereits unmittelbar nach dem Füllen abschätzen zu können, welche Flüssigkeitsmenge im Schaum gebunden ist, wird mit dem seitlich angeordneten Laser 13 ein Strahl von einigen µsec bis wenigen msec in den Schaum eingestrahlt, der den von der Seite her erkennbaren Lichtfleck 17a bzw. 17b in dem Schaum erzeugt, der je nach Dichte eine unterschiedliche Kontur hat. Die Kontur dieses Lichtflecks wird mit der seitlich zur Strahlrichtung positionierten Kamera 15 erfasst und aus dem Messergebnis dann die Flüssigkeitsmenge ermittelt, z.B. durch Vergleich mit zuvor erfassten Daten.

Die Messeinrichtung 15 wird für gewöhnlich derart konzipiert, dass sie zusätzlich zur Erfassung der Lichtflecken auch die Höhe der Schaumschicht bestimmen kann. Damit ist es zusätzlich möglich mehrere entlang der Längserstreckung der Getränkeflasche 10 angeordnete Laser für die Bestimmung der Schaumdichte mit einzuschließen. Mit einer Messeinrichtung 15 sind somit eine größere Anzahl an auf den Schaum projizierten Lichtflecken messbar. Aus dem vertikalen Schaumdichteprofil und der Schaumhöhe läßt sich bei bekanntem Innendurchmesser die im Schaum gespeicherte Flüssigkeitsmenge per Integration berechnen.

Es reicht in der Regel aus, mit einem einzigen Messvorgang die Schaumdichte und damit die gebundene Flüssigkeitsmenge zu bestimmen. Ein Messvorgang entspricht einem Bild der Kamera. Der/die Laser sind derart getriggert, dass ihre Frequenz mit der Aufnahmezeit der Kamera korrespondiert. Alternativ können auch mehrere Bilder von den Lichtflecken erstellt werden, um beispielsweise einen zeitlichen Verlauf der Setzung des Schaums zu bestimmen.

In einer weiteren Anwendung der Erfindung wird das erfindungsgemässe System als Kontrollsystem in Verbindung mit HDE- Verfahren bei der Abfüllung von Sauerstoffempfindlichen Getränken dazu verwendet, zu überwachen, ob ausreichend Schaum erzeugt wurde, um den Restsauerstoff aus der Mündungsöffnung auszudrücken.

Die Vorrichtung kann leicht in bestehende Füllhöhenkontrolleinrichtungen integriert werden. Da die Füllhöhenkontrolle üblicherweise jetzt schon mit einem Kamerasystem durchgeführt wird, kann die Erfindung einfach umgesetzt werden, weil das bestehende System lediglich um den oder die Laser und ein geeignete Bildauswertungssoftware ergänzt werden muss.

## Patentansprüche

1. Verfahren zur Bestimmung der Schaumdichte von Schaum, der sich z.B. unmittelbar nach dem Einfüllen einer Flüssigkeit in ein Behältnis (10) bildet oder vor dem anschließenden Verschließen des Behälters erzeugt wird , wobei in den Schaum ein Lichtstrahl (14) eingestrahlt wird, **dadurch gekennzeichnet, dass** die Kontur eines vom Lichtstrahl (14) in dem Schaum erzeugten Lichtflecks (17a, 17b)
von außerhalb des Behältnisses (10) bestimmt wird und mit abgespeicherten Daten verglichen wird.

2. Verfahren nach Anspruch 1, **dadurch gekennzeichnet, dass** die Dichte des Schaums mittels mehrerer Lichtstrahlen (14), insbesondere Lichtbündel, bestimmt wird.

3. Verfahren nach Anspruch 1 oder 2, **dadurch gekennzeichnet, dass** die Kontur von durch die Lichtstrahlen (14) hervorgerufenen Lichtflecken mittels wenigstens einer Messeinrichtung (15) bestimmt wird.

4. Vorrichtung zur Bestimmung der Schaumdichte von Schaum, der sich unmittelbar nach dem Einfüllen einer Flüssigkeit in ein Behältnis (10) bildet oder vor dem anschließenden Verschließen des Behälters erzeugt wird, wobei seitlich im Bereich der Behältnisse (10) eine Lichtquelle (13) und eine Messeinrichtung (15) angeordnet sind und die Lichtquelle einen Lichtstrahl (14) in den Schaum emittiert, wobei die Messeinrichtung (15) so ausgebildet ist, dass sie die Kontur eines im Schaum vom Lichtstrahl erzeugten Lichtflecks (17a, 17b) bestimmen kann, und **gekennzeichnet durch** einen Rechner (16) konfiguriert zur Berechnung der in dem Schaum gebundenen Flüssigkeitsmenge **durch** Vergleich von gespeicherten und **durch** die Messeinrichtung (15) gemessenen Daten der Kontur des Lichtflecks (17a, 17b).

5. Vorrichtung nach Anspruch 4, **dadurch gekennzeichnet, dass** die Messeinrichtung (15), die insbesondere eine Kamera ist, seitlich vom Lichtstrahleingang (14) angeordnet ist, um den Lichtfleck (17a, 17b) von der Seite her zu messen.

6. Vorrichtung nach Anspruch 4 oder 5, **dadurch gekennzeichnet, dass** der Lichtstrahl (14) von einem Laser (13) oder einer ähnlich fokussierten Lichtquelle erzeugt wird.

7. Vorrichtung nach wenigstens einem der vorhergehenden Ansprüche 4 bis 6, **dadurch gekennzeichnet, dass** mehrere Lichtquellen (13) und Messeinrichtungen (15) seitlich zu dem Behältnis angeordnet sind.

8. Vorrichtung nach wenigstens einem der vorherigen Ansprüche 4 bis 7, **dadurch gekennzeichnet, dass** der Rechner einen Speicher aufweist zur Speicherung von Daten über das Behältnis (10) und die Flüssigkeit, insbesondere Abmessung und Materialstärke des Behältnisses (10), vorzugsweise des Flaschenhalses (11), sowie Eigenschaft der Flüssigkeit.

9. Vorrichtung nach wenigstens einem der vorhergehenden Ansprüche 4 bis 8 zum Einbau in eine bestehende Kamera-Füllhöhenkontroll-Vorrichtung.

## Claims

1. Method for determining the foam density of foam which is formed e.g.
directly after a liquid has been filled into a container (10) or before the subsequent closing of the container, where a light beam (14) is irradiated into the foam, **characterized in that** the contour of a light spot (17a, 17b) generated in the foam by the light beam (14) is determined from outside the container (10) and compared to stored data.

2. Method according to claim 1, **characterized in that** the density of the foam is determined by means of several light beams (14), in particular light bundles.

3. Method according to claim 1 or 2, **characterized in that** the contour of light spots brought about by the light beams (14) is determined by means of at least one measuring device (15).

4. Device for determining the foam density of foam which is formed directly after a liquid has been filled into a container (10) or before the container is subsequently closed, wherein a light source (13) and a measuring device (15) are disposed laterally in the area of the containers (10), and the light source emits a light beam (14) into the foam, wherein the measuring device (15) is designed such that it can detect the contour of a light spot (17a, 17b) generated in the foam by the light beam, and **characterized by** a computer (16) configured for calculating the liquid quantity bound in the foam by comparing stored data to data of the contour of the light spot (17a, 17b) measured by the measuring device (15).

5. Device according to claim 4, **characterized in that** the measuring device (15), which is in particular a camera, is disposed laterally to the light beam input (14) to measure the light spot (17a, 17b) from the side.

6. Device according to claim 4 or 5, **characterized in that** the light beam (14) is generated by a laser (13) or a similarly focused light source.

7. Device according to at least one of the preceding claims 4 to 6, **characterized in that** several light sources (13) and measuring devices (15) are disposed laterally to the container.

8. Device according to at least one of the preceding claims 4 to 7, **characterized in that** the computer comprises a memory for storing data on the container (10) and the liquid, in particular the dimension and material thickness of the container (10), preferably of the neck of the bottle (11), as well as property of the liquid.

9. Device according to at least one of the preceding claims 4 to 8 to be installed into an existing camera filling level control device.

## Revendications

1. Procédé pour définir la densité d'une mousse qui se forme par exemple juste après le déversement d'un liquide dans un récipient (10) ou avant la fermeture du récipient qui suit, étant précisé qu'un rayon lumineux (14) est introduit dans la mousse, **caractérisé en ce que** le contour d'une tache lumineuse (17a, 17b) produite dans la mousse par le rayon lumineux (14) est défini de l'extérieur du récipient (10) et est comparé à des données mises en mémoire.

2. Procédé selon la revendication 1, **caractérisé en ce que** la densité de la mousse est définie à l'aide de plusieurs rayons lumineux (14), en particulier de plusieurs faisceaux lumineux.

3. Procédé selon la revendication 1 ou 2, **caractérisé en ce que** le contour de taches lumineuses provoquées par les rayons lumineux (14) est défini à l'aide d'au moins un dispositif de mesure (15).

4. Dispositif pour définir la densité d'une mousse qui se forme juste après le déversement d'un liquide dans un récipient (10) ou avant la fermeture du récipient qui suit, étant précisé qu'une source lumineuse (13) ou un dispositif de mesure (15) sont disposés sur le côté dans la zone des récipients (10) et que la source lumineuse émet un rayon lumineux (14) dans la mousse, étant précisé que le dispositif de mesure (15) est conçu pour pouvoir définir le contour d'une tache lumineuse (17a, 17b) produite par le rayon lumineux dans la mousse, et **caractérisé par** un ordinateur (16) qui est configuré pour le calcul de la quantité de liquide fixée dans la mousse, en comparant des données mises en mémoire et des données du contour de la tache lumineuse (17a, 17b) mesurées par le dispositif de mesure (15) .

5. Dispositif selon la revendication 4, **caractérisé en ce que** le dispositif de mesure (15), qui est constitué en particulier par une caméra, est disposé sur le côté de
l'entrée de rayon lumineux (14) afin de mesurer la tache lumineuse (17a, 17b) à partir du côté.

6. Dispositif selon la revendication 4 ou 5, **caractérisé en ce que** le rayon lumineux (14) est produit par un laser (13) ou une source lumineuse focalisée d'une manière similaire.

7. Dispositif selon l'une des revendications 4 à 6 précédentes, **caractérisé en ce que** plusieurs sources lumineuses (13) et dispositifs de mesure (15) sont disposés sur le côté du récipient.

8. Dispositif selon l'une des revendications 4 à 7 précédentes, **caractérisé en ce que** l'ordinateur comporte une mémoire pour stocker des données concernant le récipient (10) et le liquide, en particulier les dimensions et l'épaisseur de matériau du récipient (10), de préférence du goulot de bouteille (11), et la caractéristique du liquide.

9. Dispositif selon l'une des revendications 4 à 8 précédentes, à monter dans un dispositif à caméra de contrôle de niveau de remplissage existant.
